# EUROPEAN PATENT SPECIFICATION

(11) **EP 2 522 424 B1**
(45) Date of publication and mention of the grant of the patent: **08.01.2020**
(21) Application number: 11165275.6
(22) Date of filing: 09.05.2011
(51) Int. Cl.: B01J 29/06, B01J 35/02, C07C 2/12

(54) **SUPPORTED NANOCATALYST FOR CONVERSION OF MONOOLEFINS, PROCESS FOR CONVERSION OF MONOOLEFINS AND PROCESS FOR PREPARING THE NANOCATALYST**
GESTÜTZTER NANOKATALYSATOR ZUR UMWANDLUNG VON MONOOLEFINEN, UMWANDLUNGSVERFAHREN VON MONOOLEFINEN UND VERFAHREN ZUR HERSTELLUNG DES NANOKATALYSATORS
NANO-CATALYSEUR SUPPORTÉ POUR LA CONVERSION DE MONO-OLÉFINES, PROCÉDÉ DE CONVERSION DE MONO-OLÉFINES ET PROCÉDÉ DE PRÉPARATION DU NANO-CATALYSEUR

(43) Date of publication of application: 14.11.2012
(73) Proprietor: King Abdulaziz City for Science and Technology, Riyadh 11442 (SA)
(72) Inventor: Al-Kinany, Mohammed Chafik, 11442 Riyadh (SA); Al-Khowaiter, Soliman, 11442 Riyadh (SA); Al-Drees, Saud, 11442 Riyadh (SA); Alshehry, Feras, 11442 Riyadh (SA); Al-Rasheed, Rasheed, 11442 Riyadh (SA)
(74) Representative: Goddar, Heinz J.

(56) References cited:
- GB-A- 1 205 512
- US-A- 4 029 719
- US-A- 4 551 438
- US-A- 4 608 450
- US-A- 5 108 970

## Description

The present invention relates to a supported nanocatalyst for the conversion of monoolefins, a process for conversion of monoolefins and a process for preparing the nanocatalyst. The conversion of monoolefins is preferably a dimerization of C2 - C5 olefins to produce additives which could be used for gasoline and jet fuel to raise octane number and to improve the combustion properties.

Oxygenates are hydrocarbons that contain one or more oxygen atoms. The primary oxygenates are alcohols and ethers, including: fuel ethanol, methyl tertiary butyl ether (MTBE), ethyl tertiary butyl ether (ETBE), and tertiary amyl methyl ether (TAME).

The extent of blending of MTBE into motor gasoline increased dramatically since it was first produced 20 years ago. MTBE has a blending octane number in the range of 106-110 and make up approximately 10-11% of the gasoline pool. MTBE usage grew in the early 1980s in response to octane demand resulting initially from the phase out of lead from gasoline and later from rising demand for premium gasoline.

Methyl tertiary butyl ether use was mandated into United States automobile gasoline in the early 1990s. Due to leaking storage and distribution systems, MTBE contamination of water supplies has been discovered, causing the State of California to completely ban MTBE in 2005. The US congress is currently debating bills that will likely ban MTBE throughout the US, refiners will replace MTBE in gasoline with ethanol, alkylate, isooctane, and reformate. Some MTBE plants shut down and some will be converted to isooctane or alkylation processes in order to consume the excess isobutylene no longer consumed for MTBE.

In view of MTBE phase out schedules adopted, various options for octane enhancement have been explored. The following main oil refining processes play a key role in gasoline production:
- Crude oil distillation
- Catalytic cracking conversion processes
- Upgrading processes, such as catalytic reforming, isomerization, alkylation, polymerization of light olefins to form polygasoline, etherification processes, and isobutene dimerization

The alkylation of isobutane with light olefins is the most important refining process for the production of high octane RON and MON compounds, with low Reid vapor pressure. At present the demand for alkylates is growing due to environmental concerns about reducing aromatics in the gasoline pool. This can be made up for by using clean-burning alkylate compounds. In this sense, gasoline from alkylation of isobutane with light olefins is the preferred blending component for reformulated gasoline, since alkylate is a clean burning fuel and is mainly formed by low reactive isoalkanes. Also, environmental concerns over MTBE and its expected removal from the gasoline pool will require further increase of alkylates.

Traditionally, liquid acids such as H₂SO₄ and HF have been used in industry to catalyse alkylation reactions. Both of H₂SO₄ and HF suffer from certain drawbacks. Anhydrous HF is corrosive and highly toxic. If HF is released into atmosphere, it forms stable aerosols, which drift down and spread on the ground for several kilometres. For that reasons refineries with HF alkylation plants are under pressure to install expensive mitigation systems minimizing the degree of HF leaks. Moreover, authorities in many industrialized countries cease to license new HF alkylation plants.

H₂SO₄ also is a corrosive liquid but not volatile, making its handling easier. Its major disadvantage is the acid consumption, which can be as high as 70 - 100 kg acid/ton alkylate, very low hydrocarbon solubility, and optimum reaction temperature of 4 - 10°C.

Therefore, refrigeration is necessary to remove process heat. A temperature higher than 18°C leads to oxidation of hydrocarbons forming SO₂ and water. The spent acid contains water and heavy hydrocarbons and has to be regenerated, usually by burning.

Isooctane or branched isomers of octane could be produced by alkylation of isobutane with isobutene. This process produces trimethylpentanes and dimethylhexanes. Branched octanes could also be produced from dimerization of isobutene as cited in the following patents: EP 0994088, GB 2325237, and US 6011191.

Tertiary alkyl ethers and branched hydrocarbons also could be produced by etherification of aliphatic alcohols with isoolefins as cited in the following patents: US 5,637,777; 5,908,964; 5,536,886 and 6,369,280.Therefore, most research in this area, at present, is moving towards finding alternative materials as additives to raise the octane number of gasoline and to improve its combustion properties. Also, the research is concerning in developing new catalysts to produce clean fuels free of sulfur, nitrogen and aromatic compounds with low volatility and high octane number.

Based on the foregoing, an alternative method to produce additives with high octane number is dimerization of low olefins or mixtures of olefins. The alkylates produced from the dimerization are ideal for reforming gasoline.

The reformulated gasoline will meet the requirements of environmental laws in the future because it is characterized by high octane number and low volatility and is completely free of sulphur, nitrogen and aromatic compounds.

Based on this, industries and research and development centres have been researching and developing solid acid catalyst in the past decade.

Isobutene dimers and oligomers are especially useful intermediates for preparation of various products of commercial interest, such as isoparaffms, higher alcohols, aldehydes, or carboxylic acids having 8, 12 or 16 carbon atoms. Amongst such products, highly branched trimethylpentanes that can be obtained through hydrogenation of trimethylpentenes are of particular interest as gasoline octane number enhancers. Indeed, trimethylpentanes have high RONs (Research Octane Numbers) of 100 and MONs (Motor Octane Number) of 100.

Therefore, dimerization of olefins especially isobutene is a commercially important reaction. The process is carried out by batch or continuous process, either in the gas or liquid phase, generally at temperatures ranging from 50 - 280°C and under atmospheric pressures or under pressures which are such as to keep the reactants in liquid phase, if desired.

Many catalysts have been claimed for dimerization and oligomerization of olefins, such as acids, cation exchange acid resins, silico-aluminas, mixed oxides, zeolites, fluorinated or chlorinated aluminas, and many other catalysts.

The oligomerization process using supported phosphoric acid on kieselgur as catalyst has been used for several years to produce gasoline. The reaction is carried out at temperatures over 200°C, and products range from the dimer (C₈) to higher polymeric olefins (C₁₆). In the past other processes have used various catalysts for converting the isobutene to C₈ dimer. For example, a process using a molecular sieve at elevated temperature is disclosed in U.S. 3,531,539. U.S. 4,215,100 discloses the use of an acid cation exchange resin in a heterogeneous combination reaction/distillation system for the selective dimerization of isobutene in the presence of normal butenes.

The scientific interest and commercial importance of butenes dimerization have led to the search for new solid catalytic materials that can avoid the formation of higher molecular weight olefins due to their controlled acidity. Among the catalysts reported for this reaction, other than supported phosphoric acid on kieselgur, are: Ziegler - Natta based catalysts, sulfonic resins, benzyl sulfonic acid on silica, mica montmorillonite, zirconium oxide, and a variety of other catalysts have been claimed in US. 3,760,026.

When dimerizing olefins, it is known that heavy oligomers are generated as by products, which gradually deactivate the catalyst. U.S 30088134 relates to a process for dimerizing isobutene, the catalyst employed in this case is a porous cation exchange resin comprising a styrene polymer, which is cross- linked with divinylbenzene, and any sulphonic acid groups adhering to the polymer.

US 4,551,438 relates to a catalyst composition for promoting oligomerisation of olefins, said composition comprising the components: a) nickel-containing silicaceous crystalline molecular sieves in the hydrogen form selected from the group consisting of ZSM-5, ZSM-11, crystalline mixtures of ZSM-5 and ZSM-11, silicalite, an organic silicate disclosed in Ser.No.Re. 29,948 and CZM or mixtures thereof; and b) at least one hydrocarbyl aluminium halide; the molar ratio of the hydrocarbyl aluminium halide to the nickel being from 1:1 to 10:1.

GB 1,205,512 relates to a process for the production of dimers of olefins comprising a relatively high proportion of linear dimers, which process comprises dimerizing an olefin at an elevated temperature below 300°C, in contact with a zeolite X-type catalyst, derived from an alkali metal-based X-type zeolite, wherein 30 - 60% of available cations initially present in the zeolite are exchanged for nickel ions and 1 - 30% are exchanged for calcium, cadmium, zinc or manganese ions, the maximum total ion exchange being 80%.

US 4,029,719 relates to a process for the preparation of linear olefins having from 6 to 24 carbon atoms in the molecule, characterised by the introduction of one or more linear olefins having from 3 to 12 carbon atoms in the molecule to the catalyst obtained by activating, by means of an organic or inorganic base, natural or synthetic crystalline zeolite on which has previously been deposited by ion exchange at least one metal chosen from among those belonging to group VIII of the periodic system of elements in an amount of from 0.1% to 50% by weight with respect to the zeolite, bringing the resulting product into contact with an organic or inorganic base in the liquid or gaseous form at a temperature in the range from -35 to 250°C for a time of at least 30 min., eliminating the excess of the base and heat treating at temperatures in the range from 50 to 450°C for at least 30 min., and by operating at temperatures of from 130° to 260°C at a pressure of from 2 to 70 atm. US 5,108,970 relates to a catalyst composition consisting of a zeolite containing an amount of nickel effective for oligomerisation of C₃-C₁₂ olefin, which catalyst is prepared by a process consisting of (1) adding nickel in the form of nickel chloride or nickel nitrate to the zeolite by cation exchange or impregnation, the zeolite being selected from the group consisting of cesium or barium exchanged (CSZ-1) high silica ultra-stable faujasite and zeolites zeolite isostructural with mazzite, and (2) vacuum calcining said nickel containing zeolite composition at a temperature of about 375° C to 475° C.

US 4,608,450 relates to a process for preparing olefinic tetramer comprising: a) contacting in a first stage a C₃ or C₄ olefin stream or mixture thereof, in the liquid state with an Ni-HZM-5 catalyst at a temperature from about 80° to 200° F and a pressure of about 400 to 1600 psig to produce a first effluent containing at least 70% by weight dimer of said C3 or C4 olefin; and b) contacting in a second stage the first effluent in the liquid state with a second Ni-HZM-5 catalyst at a temperature of from about 250° to 450° F and a pressure of 200 to 800 psig to produce a second effluent containing at least 60% tetramer of said C₃ or C₄ olefin or mixture thereof.

The main problem of dimerization, which has hindered its industrial development, is the difficulty in controlling the reaction rate, the activity of all acid catalysts together with the difficulty in controlling the temperature in the reactor which makes it extremely difficult to succeed in limiting the addition reactions of isobutene to growing chains and consequently to obtain a high quality product characterized by a high selectivity to dimers.

In dimerization reactions there is the formation of excessive percentages of heavy oligomers such as trimers (selectivity of 15- 60%) and tetramers (selectivity of 2- 10%) of isobutene. Tetramers are completely outside the fuel fraction as they are too highly- boiling and therefore present a net loss in yield of fuel; as far as trimers are concerned, their concentration should be significantly decreased as they are characterized by a boiling point (170- 180°C) at the limit of future specifications on the final point of reformulated fuels.

The problems resulting from the prior art are solved in accordance with the subject matter of the independent claims. Preferred embodiments of the invention are described in the su-claims. It is thus an object of the present invention to provide a nanocatalyst for the conversion of monoolefins, especially for dimerization of monoolefins, preferably of C4-olefins, which overcomes the drawbacks of the prior art, wherein the catalyst shows high activity and selectivity to preferred products. Further, the nanocatalyst shall be environmentally friendly and shall provide high conversions and selectivity. Especially, using the nanocatalyst in a process for conversion of monoolefins shall result in the provision of clean fuel distillates in the range of gasoline and jet fuel, which product shall be free of sulfur, nitrogen and aromatic compounds with high octane number and Reid pressure.

It is a second object of the present invention to provide a process for the conversion of monoolefins to result in the benefits as mentioned above.

A third object relates to the provision of a process for preparing the nanocatalyst.

The first object is achieved by a supported nanocatalyst for conversion of monoolefins, comprising at least one particulate zeolite support, at least one transition metal oxide, wherein the transition metal is selected from the group consisting of Mn, Cr, V, Zr, Mo, W, Pd, Pt, Ru, Ni, Co, W and Zn, the transition metal oxide being supported on the support, wherein the average particle size of the supported nanocatalyst is from 25 - 500 nm, preferably 25 - 400 nm and pores of the nanocatalysts have an average diameter of 55 - 65 AngströmPreferably, the zeolite support has hydrogen nominal cation form and/or a SiO_{2/}Al₂O₃ molar ratio of 30. Such a zeolite is for example zeolite CBV 720 having, further, a Na₂O weight percent of 0.03, a unit cell size A of 24.28 and a surface area of 780 m²/g.

Preferably, the support additionally comprises at least one oxide of elements of the group consisting of Ba Ca, K, Mg, Sn, Si, Ga and Al.

The average particle size of the inventive nanocatalyst is preferably determined by laser diffraction. The technique of laser diffraction is well known in the art and is based on the principle that particles passing through a laser beam will scatter light at an angle that is directly related to their size. As the particle size decreases, the observed scattering angle decreases logarithmically. The observed scattering intensity is also dependent on particle sizes and diminishes, to a good approximation, in relation to the particle's cross-sectional area. Large particles therefore scatter light at narrow angles with high intensity, whereas small particles scatter at wider angles but with low intensity.

The primary measurement that has to be carried out within a laser diffraction system is the capture of the light scattering data for the particles under study. A typical system consists of a laser, a sample presentation system and a series of detectors.

The measurement of the average particle size is carried out in dry condition, i.e. after calcination and ultrasonating. For elemental analysis of the inventive nanocatalyst, X-ray fluorescence (XRF) was utilized which is one of the most widely used spectroscopic techniques in elemental identification and quantification. X-ray elemental analysis studies were recorded at 30 kV and room temperature using JEOL elemental analyzer JSX-3201.

Even preferred, the average particle size of the support is from 25 - 400 nm, preferably 25 - 150 nm.

In one preferred embodiment, the nanocatalyst comprises from 0.1 - 65% by weight, preferably 4 - 30% by weight of the transition metal oxide, based on the total weight of the support.

The BET surface area of the nanocatalyst may be 100-300 m²/g.

Measurement of the BET-surface area is well known in the art (BET=Brunauer, Emmett, Teller).

Surface area and pore size measurements studies were carried out by using micromeritics adsorption equipment of ASAP 2010. All samples were degassed under vacuum at 300°C. The adsorption of nitrogen was measured at -196°C, the surface areas were calculated using the BET method based on adsorption data.

The pore-size distribution was analyzed applying the same procedure described above from desorption branch of the isotherm by the Parrett-Joyner-Halenda method.

The second object is achieved by a process for conversion of monoolefins comprising the step of converting the monoolefins in the presence of an inventive nanocatalyst.

Preferably, the monoolefins have from 2 - 5 carbon atoms.

The process can be a dimerization of monoolefins.

Even preferred, the process is carried out continuously or batchwise.

Preferably the process is carried out in a continuous gas phase using fixed bed reactor or in a batch liquid phase in an autoclave
For a gas phase continuous process, it is convenient and preferred to work under pressures ranging from 1 - 10 atm, preferably from 1 to 8 atm, and temperatures ranging from 20 to 275°C, a preferred range being from 25 to 200°C.

The catalyst can be re-generated by means of various methods, such as heating the used catalyst in situ up to 550°C in the presence of air or oxygen.

In one embodiment, the process is carried out at a temperature range between -15 to 600°CIt is also preferred that process is carried out in the presence of inert gas.

Preferably, the process is carried out with a weight hourly space velocity of 0.1 to 40h⁻¹, preferably 0.1 to 6h⁻¹.

The third object is achieved by a process for preparing an inventive nanocatalyst, comprising the steps: a) adding at least one solution of at least one transition metal compound to a particulate zeolite support having preferably an average particle size of 15 - 400 nm, and mixing thereofto produce a paste, b) drying the pasteand then calcining it ,and c) ultrasonating the catalyst obtained in step (b), preferably for 12 hours.

It may happen that agglomeration occurs to some extent when preparing the inventive supported nanocatalyst according to steps (a) - (b). Agglomeration can be avoided by ultrasonating the nanocatalyst obtained in step (b).

Finally mixing in step a) is ultrasonating, preferably for 1 - 12 hours.

It was surprisingly found that the inventive nanocatalyst based on a zeolite support can be successfully utilized in the conversion, especially dimerization, of monoolefins, preferably C₄-monoolefins. The use of the catalyst results in the provision of clean fuel distillates in the range of gasoline and jet fuel which are free of sulfur, nitrogen and aromatic compounds. Further, it was found that the catalyst shows a high activity and a long life time, wherein the catalyst can be reactivated and reused which makes it environmentally friendly. The conversion of monoolefins and fuel distillates reached up to 95% under economical operational conditions of temperature, pressure and space velocity. Especially the conversion can be achieved with space velocities ranging from 0.1 - 40h⁻¹.

The process for conversion of monoolefins is most preferably the dimerization of isobutene to produce branched Cg alkylates which can be used as additives for gasoline and jet fuel, especially for improving the combustion properties of gasoline.

The supported nanocatalyst according to the invention is remarkable stable and active for long periods of time. Further, the level of catalytic activity exhibited is normally substantially higher than that of the corresponding homogeneous catalyst, such as sulfuric acid, hydrofluoric acid and phosphoric acid.

Preferably the transition metal is selected from Co, Mn, Cr, V, Zr, Mo, W, and Pd, or mixtures thereof. If in a preferred embodiment amongst the zeolite support additional oxides are present, these oxides are preferably a mixture of Al₂O₃ and SiO₂.

Particle size of the support of the supported nanocatalyst is in a range from 25 to 500 nm and may be preferably in the form of powder, pellets, granules, or any other form.

Reactants applicable for use in the process for the conversion of monoolefins are monoolefinic compounds which can (a) self-react, i.e. especially dimerize, (b) react with other olefinic compounds, i.e. especially co-dimerize; and (c) react with paraffinic compounds. Examples are ethylene, propylene, 1-butene, cis-2-butene and isobutylene. Preferably the olefins have four carbon atoms. Paraffinic compounds may be butane and isobutane.

In a preferred dimerization of isobutene, the formation of heavy oligomers, such as trimers and tetramers, occurs. At virtually 95% conversion, the yield of branched C8 isomers is from 58 to 65%. In addition, from 5 to 35% of higher oligomers is obtained, with about 5 - 20% trimers and 1 - 5% tetramers.

Concentration of oligomers can be limited by a selection of operating parameters such as temperature, pressure or space velocities. A further possibility is to keep the concentration of reactants at the reactor inlet below 90%, preferably by addition of inert gas.

The nanocatalyst for conversion of monoolefins can be produced based on an ultrasonic vibration technique, comprising the following steps:
i. Preparing at least one aqueous solution of a water-soluble metal salt selected from one of the transition metal elements as disclosed above for the catalytically active component of the catalyst.
ii. Ultrasonating the at least one aqueous solution for about 1 to 5 hours to obtain a homogenous solution.
iii. Adding the at least one active solution to a nanoparticle support as disclosed above and ultrasonating the mixture for about 1 to 12 hours. Preferably, the zeolite has the nominal cation form of hydrogen and a molar ratio SiO₂Al₂O₃ of 30, such as zeolite CBV 720.
iv. Drying the produced paste and calcining it initially in an inert atmosphere and finally in an oxidizing atmosphere.
v. Ultrasonating the produced nanocatalyst under dry conditions.

The transition metal salts for step (i) are preferably nitrate, acetate or chloride. An amount of metal salt is employed to result in a transition metal oxide weight percentage of preferably 0.1 to 65 weight percent, based on the weight of the zeolite.

Preparation of the solution and ultrasonating thereof in step (iii) are conveniently carried out at temperatures ranging between 25 to 80°C. Ultrasonating in step (v) can be carried out at a temperature of 25 to 60°C, preferably 40°C.

The drying step (iv) is conveniently carried out at a temperature below 100°Cunder vacuum, for a time sufficient to remove water completely. The calcination of step (iv) is carried out at an atmosphere which is inert (such as helium) and then oxidizing (such as air or oxygen)

The supported nanocatalyst thus obtained must be ultrasonated under dry conditions to disintegrate agglomerated particles. The final particle size of the nanocatalyst is then in a range from 25 - 500 nm.

The supported nanocatalyst thus obtained is ultrasonated under dry conditions in order to avoid the possible formation of agglomerates. Zetasizer nanoparticle analyzer series fitted with a 633 nm "red" laser was used for particle size measurement. Ultrasonic homogenizer was used to dispose and to deagglomerate the calcined supported nanocatalyst into solvent such as methanol, ethanol, isopropanol, isobutanol, and water, or mixtures thereof.

The following examples describe the preparation of nanocatalyst and dimerization, or codi-merization, of olefin or a mixture of olefins to produce branched isomers of Cg.

### Example 1

### Preparation of nanocatalyst

(i) Solution (A): 4.91 g (3.97 mmol) of ammonium molybdate(VI)tetrahydrate, (Aldrich chemicals, 99.0% pure), or any of the following salts of the transition metals could be used; manganese(II) acetate, manganese(II) chloride, manganese(II)nitrate, ammonium metavanidate, vanadium(III) chloride or vanadium(III) acetylacetonate, molybdenum(II) acetate dimer or molybdenum(II)chloride, was dissolved at room temperature in 100 ml of deionized water. The aqueous solution is stirred at room temperature for 60 minutes, then at 50°C for 2 hours until a clear solution is obtained. The resulting solution is filtered and then ultrasonated for 60 minutes.
(ii) Solution (B): 4.94 g (16.97 mmol) of cobalt nitrate hexahydrate, (Aldrich chemicals, 99.2% pure), or any of the following salts of the transition metals could be used; cobalt(II)acetate tetrahydrate, cobalt(II)chloride, chromium(III)acetate monohydrate, chromium(II)chloride or chromium(III) nitrate nonahydrate was dissolved at room temperature in 100 ml of deionized water. The aqueous solution is stirred at room temperature for 60 minutes, and then at 50°C for 2 hours until a clear solution is obtained. The resulting solution is filtered and then ultrasonated for 60 minutes.
(iii) The solutions (A) and (B) are combined, and the mixture obtained is stirred at 50°C for 2 hours until a homogeneous clear solution is obtained which is ultrasonated for 3 hours at 80°C.
(iv) The heated ultrasonated mixture of (iii) is added to 11.82 g of Zeolite support with hydrogen nominal cation form with 30 mole ratio of SiO₂/Al₂O₃ (Zeolyst International), or any of the following inert supports could be additionally used; MgO, K₂O, SiO₂ or mixtures thereof. The resultant mixture is stirred for 60 minutes and ultrasonated for 12 hours at temperatures of 30-50°C until a homogeneous mixture is obtained.
(v) The resulted mixture from (iv) is dried using a rotary evaporator under vacuum at a temperature in the range of 70 to 90°C, and then in a programmable oven under vacuum at temperatures ranging from 25 to 95°C. The mixture is then calcined at programmable temperature of 25 to 700°C, preferably from 25 to 550°C, first in helium for 1 hour, and then in air or oxygen stream for 12 hours.
(vi) The produced supported catalyst is then ultrasonated under dry conditions for 3h. Zetasizer nono-particle analyzer series fitted with a 633 nm "red" laser was used for particle size measurement. Ultrasonic homogenizer was used to dispose and deagglomerate the calcined supported catalyst into solvent such as methanol, ethanol, isopropanol, isobutanol, and water, or mixtures thereof.

### Example 2

(i) 4.91 g (3.97 mmol) of ammonium molybdate(VI)tetrahydrate, (Aldrich chemicals, 99.0% pure), or any of the following salts of the transition metals could be additionally used; manganese(II) acetate, manganese(II) chloride, manganese(II)nitrate, ammonium metavanidate, vanadium(III) chloride or vanadium(III) acetylacetonate, molybdenum(II) acetate dimer or molybdenum(II)chloride, was dissolved at room temperature in 100 ml of deionized water. The aqueous solution is stirred at room temperature for 60 minutes, then at 50°C for 2 hours until a clear solution is obtained. The resulting solution is filtered and then ultrasonated for 60 minutes.
(ii) Solution of (i) is added to 11.82 g of Zeolite support with hydrogen nominal cation form (Zeolyst International), or any of the following inert supports could be additionally used; MgO, K₂O, SiO₂ or mixtures thereof. The resultant mixture is stirred for 60 minutes and ultrasonated for 12 hours at temperatures of 30-50°C until a homogeneous mixture is obtained.
(iii) The resulted mixture from (ii) is dried using a rotary evaporator under vacuum at a temperature in the range of 70 to 90°C, and then in a programmable oven under vacuum at temperatures ranging from 25 to 95°C. The mixture is then calcined at programmable temperature of 25 to 700°C, preferably from 25 to 550°C, first in helium for 1 hour, and then in air or oxygen stream for 12 hours.
(iv) The produced supported catalyst is then ultrasonated under dry conditions for 3h. Zetasizer nono-particle analyzer series fitted with a 633 nm "red" laser was used for particle size measurement. Ultrasonic homogenizer was used to dispose and deagglomerate the calcined supported catalyst into solvent such as methanol, ethanol, isopropanol, isobutanol, and water, or mixtures thereof.

A supported metal oxide or oxides nanocatalyst is obtained with a quantitative yield relative to the initially charged material, said powder with 25 - 500 nm particles size.

### Example 3

### Dimerization of olefin or a mixture of olefins using catalyst prepared in example(1)

Typically, in a gas phase continuous process, olefin such as isobutene or a mixture of olefins (1:1 mole ratio) such as isobutene with *cis*-2-butene, *trans*-2-butene, 1-butene, propene, or isopentene was processed in a down flow fixed bed tube reactor over supported metal oxides nanocatalyst. Isobutene as a feedstock is preferred.

The process was performed using the catalyst prepared in example (1) in a gas phase using a fixed bed reactor at temperatures ranging from 20°C to 275°C, wherein temperatures of about 50°C to about 150°C are preferred. In a liquid phase batch process, the temperature ranging from -5°C to 250°C is preferred.

The reaction pressure can vary depending on the feed employed. The processing in this example was performed at pressure ranging from 1 to about 10 atm. Preferably, pressures of atmospheric to about 4 atm are employed. In a liquid phase batch process the pressure of 1 to about 15 atm are preferred.

The contact time required for the reaction depends on the feed and the reaction conditions. A space velocity (WHSV) of about 0.1 to about 25 h⁻¹ could be employed. A WHSV of about 0.1 to 5 h⁻¹ is preferred for isobutene feedstock.

Analysis of the hydrocarbons achieved from dimerization is performed using gas chromatography instrument fitted with a flame ionisation detector (FID). The column used in all analysis quoted was a (100mX 0.25 mm) glass open tubular capillary column. The column temperature was programmed as an initial temperature of 30°C for 15 min, then 60°C for 20 min (heating rate 1°C/min) and finally 200°C for 20 min (heating rate 2°C/min).

The FID detector temperature was 250°C; injector 300°C. The maximum column temperature at which the stationary phase is stable is 200°C.

Identification of dimerization products was performed on GC/MS fitted with poina 50 metres glass open tubular capillary column. The column temperature was programmed as described above.

### Example 4

### Dimerization of olefin or a mixture of olefins using catalyst prepared in example(2)

Typically, in a gas phase continuous process, olefin such as isobutene or a mixture of olefins (1:1 mole ratio) such as isobutene with *cis-*2-butene, *trans*-2-butene, 1-butene, propene, or isopentene was processed in a down flow fixed bed tube reactor over supported metal oxides nanocatalyst. Isobutene as a feedstock is preferred.

The process was performed using the catalyst prepared in example (2) in a gas phase using fixed bed reactor at temperatures ranging from 20°C to 275°C, wherein temperatures of about 50°C to about 150°C are preferred. In liquid phase batch process, the temperature ranging from -5°C to 250°C is preferred.

The reaction pressure can vary depending on the feed employed. The processing in this example was performed at pressure ranging from 1 to about 10 atm. Preferably, pressures of atmospheric to about 4 atm are employed. In liquid phase batch process the pressure of 1 to about 15 atm are preferred.

The contact time required for the reaction depends on the feed and the reaction conditions. A space velocity (WHSV) of about 0.1 to about 25 h⁻¹ could be employed. A WHSV of about 0.1 to 5 h⁻¹ is preferred for isobutene feedstock.

The following table shows the reaction conditions of dimerization and the product distribution with the conversion of feed stocks.

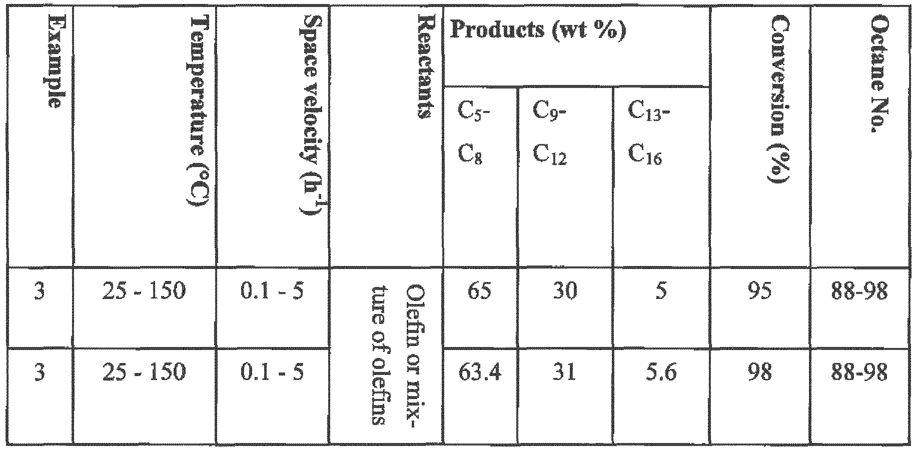

As can be seen, the conversion of monoolefins reached up to 98% with the yield up to 65% to branch alkylates under economical operation conditions of temperature, pressure and space velocity. The octane number of the products ranges between 88 - 98, and the Reid pressure ranges between 5 - 250 Pa.

## Claims

1. Supported nanocatalyst for conversion of monoolefins, comprising:
(i) at least one particulate zeolite support,
(ii) at least one transition metal oxide, wherein the transition metal is selected from the group consisting of Mn, Cr, V, Zr, Mo, W, Pd, Pt, Ru, Ni, Co, W and Zn, the transition metal oxide being supported on the support,
wherein the average particle size of the supported nanocatalyst is from 25 - 500 nm, and
pores of the nanocatalyst have an average diameter of 55 - 65 Angström.

2. Nanocatalyst according to claim 1, wherein the support additionally comprises at least one oxide of elements of the group consisting of Ba, Ca, K, Mg, Sn, Si, Ga, and Al.

3. Nanocatalyst according to claim 1 or 2, wherein the average particle size of the support is from 25 - 400 nm.

4. Nanocatalyst according to any of the preceding claims, wherein the nanocatalyst comprises from 0.1 - 65 % by weight of the transition metal oxide, based on the total weight of the support.

5. Nanocatalyst according to any of the preceding claims, wherein the BET surface area of the nanocatalyst is 100 - 300 m²/g.

6. Process for conversion of monoolefins comprising the step of converting the monoolefins in the presence of a nanocatalyst according to any of the claims 1 - 5.

7. Process according to claim 6, wherein the monoolefins have from 2 - 5 carbon atoms.

8. Process according to claim 7, wherein the process is a dimerization of monoolefins.

9. Process according to claim 6 to 8, wherein the process is carried out continuously or batchwise.

10. Process according to claim 9, wherein the process is carried out in a continuous gas phase using fixed bed reactor or in a batch liquid phase in an autoclave.

11. Process according to any of the preceding claims 6 - 10, wherein the process is carried out at a temperature range between -15 to 600°C.

12. Process according to any of the preceding claims 6 to 11, wherein the process is carried out in the presence of inert gas.

13. Process according to any of the preceding claims 6 to 12, wherein the process is carried out with a weight hourly space velocity of 0.1 to 40h⁻¹.

14. Process for preparing a nanocatalyst according to claim 1, comprising the steps:
a) adding at least one solution of at least one transition metal compound to a particulate zeolite support having an average particle size of 15 - 400 nm, and mixing thereof, to produce a paste,
b) drying the paste, and then calcining it, and
c) ultrasonating the catalyst obtained in step (b).

15. Process according to claim 14, wherein mixing in step a) is ultrasonating.

## Patentansprüche

1. Geträgerter Nanokatalysator zum Umwandeln von Monoolefinen, umfassend:
(i) zumindest einen partikulären Zeolithträger,
(ii) zumindest ein Übergangsmetalloxid, wobei das Übergangsmetall ausgewählt ist aus der Gruppe, bestehend aus Mn, Cr, V, Zr, Mo, W, Pd, Pt, Ru, Ni, Co, W und Zn, wobei das Übergangsmetalloxid auf dem Träger geträgert ist,
wobei die durchschnittliche Partikelgröße des geträgerten Nanokatalysators von 25 - 500 nm ist, und
Poren des Nanokatalysators einen durschnittlichen Durchmesser von 55 - 65 Angström haben.

2. Nanokatalysator nach Anspruch 1, wobei der Träger zusätzlich zumindest ein Oxid der Elemente der Gruppe, bestehend aus Ba, Ca, K, Mg, Sn, Si, Ga und Al, umfasst.

3. Nanokatalysator nach Anspruch 1 oder 2, wobei die durchschnittliche Partikelgröße des Trägers von 25 - 400 nm ist.

4. Nanokatalysator nach einem der vorangehenden Ansprüche, wobei der Nanokatalysator von 0,1 - 65 Gew.-% des Übergangsmetalloxids, bezogen auf das Gesamtgewicht des Trägers, umfasst.

5. Nanokatalysator nach einem der vorangehenden Ansprüche, wobei die BET-Oberflächenfläche des Nanokatalysators von 100 - 300 m²/g ist.

6. Verfahren zum Umwandeln von Monoolefinen, umfassend den Schritt des Umwandelns der Monoolefine in der Gegenwart eines Nanokatalysators nach einem der Ansprüche 1 bis 5.

7. Verfahren nach Anspruch 6, wobei die Monoolefine 2 - 5 Kohlenstoffatome haben.

8. Verfahren nach Anspruch 7, wobei das Verfahren eine Dimerisierung von Monoolefinen ist.

9. Verfahren nach einem der Ansprüche 6 bis 8, wobei das Verfahren kontinuierlich oder chargenweise durchgeführt wird.

10. Verfahren nach Anspruch 9, wobei das Verfahren in einer kontinuierlichen Gasphase unter Verwendung eines Festbettreaktors oder in einer Charge einer flüssigen Phase in einem Autoklaven durchgeführt wird.

11. Verfahren nach einem der vorangehenden Ansprüche 6 - 10, wobei das Verfahren bei einer Temperatur zwischen -15 bis 600°C durchgeführt wird.

12. Verfahren nach einem der vorangehenden Ansprüche 6 bis 11, wobei das Verfahren in Gegenwart von Inertgas durchgeführt wird.

13. Verfahren nach einem der vorangehenden Ansprüche 6 bis 12, wobei das Verfahren mit einer stündlichen Gewichtsraumgeschwindigkeit von 0,1 bis 40 h⁻¹ durchgeführt wird.

14. Verfahren zum Herstellen eines Nanokatalysators nach Anspruch 1, das die Schritte umfasst:
a) Zugeben zumindest einer Lösung zumindest einer Übergangsmetallverbindung zu einem partikulären Zeolithträger mit einer durchschnittlichen Partikelgröße von 15 - 400 nm und Mischen davon, um eine Paste herzustellen,
b) Trocknen der Paste und anschließendes Kalzinieren derselben, und
c) Ultraschallbehandeln des Katalysators, der in Schritt (b) erhalten wird.

15. Verfahren nach Anspruch 14, wobei das Mischen in Schritt a) Ultraschallbehandeln ist.

## Revendications

1. Nano-catalyseur supporté pour la conversion de mono-oléfmes, comprenant :
(i) au moins un support particulaire zéolithique,
(ii) au moins un oxyde de métal de transition, dans lequel le métal de transition est sélectionné dans le groupe constitué de Mn, Cr, V, Zr, Mo, W, Pd, Pt, Ru, Ni, Co, W et Zn, l'oxyde de métal de transition étant supporté sur le support.
la dimension particulaire moyenne du mono-catalyseur supporté varie entre 25 et 500 nm, et
les pores du nano-catalyseur ont un diamètre moyen de 55 à 65 Angström.

2. Nano-catalyseur selon la revendication 1, dans lequel le support comprend en outre au moins un oxyde des éléments du groupe constitué de Ba, Ca, K, Mg, Sn, Si, Ga et Al.

3. Nano-catalyseur selon la revendication 1 ou la revendication 2, dans lequel la dimension particulaire moyenne du support est de 25 à 400 nm.

4. Nano-catalyseur selon l'une quelconque des revendications précédentes, dans lequel le nano-catalyseur comprend de 0,1 à 65 % en poids de l'oxyde de métal de transition, par rapport au poids total du support.

5. Nano-catalyseur selon l'une quelconque des revendications précédentes, dans lequel l'aire de surface Brunauer-Emmet-Teller (BET) du nano-catalyseur est de 100 à 300 m²/g.

6. Procédé de conversion de mono-oléfines comprenant l'étape de conversion de mono-oléfines en présence d'un nano-catalyseur selon l'une quelconque des revendications 1 à 5.

7. Procédé selon la revendication 6, dans lequel les mono-oléfines contiennent 2 à 5 atomes de carbone.

8. Procédé selon la revendication 7, le procédé consistant en une dimérisation des mono-oléfines.

9. Procédé selon les revendications 6 à 8, le procédé étant réalisé continuellement ou par lots.

10. Procédé selon la revendication 9, le procédé étant réalisé en phase gazeuse en continu en utilisant un réacteur à lit fixe ou en phase liquide par lots dans un autoclave.

11. Procédé selon l'une quelconque des revendications 6 à 10, le procédé étant réalisé à une plage de température allant de -15 à 600 °C.

12. Procédé selon l'une quelconque des revendications 6 à 11, le procédé étant réalisé en présence de gaz inerte.

13. Procédé selon l'une quelconque des revendications 6 à 12, le procédé étant réalisé à une vitesse spatiale horaire en poids de 0,1 à 40 h⁻¹.

14. Procédé de préparation d'un nano-catalyseur selon la revendication 1, comprenant les étapes consistant à :
a) ajouter au moins une solution d'au moins un composé de métal de transition à un support particulaire zéolithique ayant une dimension particulaire moyenne de 15 à 400 nm, et mélanger le tout de manière à produire une pâte,
b) sécher la pâte, et la calciner ensuite, et
c) préparer le catalyseur obtenu à l'étape (b) par la technique d'ultrasonication.

15. Procédé selon la revendication 14, dans lequel le mélange à l'étape a) est préparé par la technique d'ultrasonication.
